# EUROPEAN PATENT SPECIFICATION

(11) **EP 2 755 701 B1**
(45) Date of publication and mention of the grant of the patent: **24.11.2021**
(21) Application number: 12778368.6
(22) Date of filing: 11.09.2012
(51) Int. Cl.: A61M 5/20, A61M 5/24, A61M 5/32

(54) **INJECTOR ASSEMBLY**
INJEKTORANORDNUNG
ENSEMBLE INJECTEUR

(30) Priority: 12.09.2011 GB 201115650; 21.11.2011 US 201161562012 P
(43) Date of publication of application: 23.07.2014
(73) Proprietor: Owen Mumford Limited, Oxford, Oxfordshire OX20 1TU (GB)
(72) Inventor: BICKNELL, Stephen, Glasgow G12 0NQ (GB); DIX, Robert, Oxford Oxfordshire OX29 5SD (GB)
(74) Representative: Lind, Robert
(86) International application number: PCT/GB2012/052236
(87) International publication number: WO 2013/038166

(56) References cited:
- US-A- 5 154 698
- US-A- 5 201 721
- US-A1- 2005 013 550
- US-B1- 6 210 369
- None

## Description

This invention relates to injection devices and in particular to autoinjector assemblies.

For reasons of safety, injection devices, such as autoinjectors, are normally provided with caps that fit over the end of the device from which the needle extends. Conventionally, such a cap is releasably connected to the main body of the autoinjector by means of sets of dimples on the inner surface of the cap that fit into corresponding recesses on the main body. However, such a conventional arrangement does not provide a particularly strong connection, meaning that the cap can be removed accidentally relatively easily. Also, as the dimples are on the inner surface of the cap, they are not visible, meaning that the user is unaware of whether or not they have correctly engaged the recesses. Such formations can also trap dirt or bacteria, leading to hygiene risks.

US 5,154,698 discloses a single use syringe having an externally longitudinally sliding conforming protection cap for the needle.

Embodiments of the present invention are intended to address at least some of the problems discussed above.

Accordingly, in one aspect, this invention provides an injector assembly including:
a main body having a first connecting arrangement, and
a cap having a second connecting arrangement for, in use, releasably connecting the cap to the first connecting arrangement of the main body,
wherein one of the first or the second connecting arrangement includes a cut-out portion having an open neck portion leading to a wider locating portion, and
wherein another of the first or the second connecting arrangement includes a projection having a neck portion leading to a wider head portion, with dimensions of the neck portion and the head portion corresponding to dimensions of the open neck portion and the locating portion so that, in use, the first and the second connecting portions releasably interlock.

At least part of the locating portion may have a maximum width greater than a maximum width of the open neck portion, and the head portion may have a maximum width greater than the maximum width of the open neck portion.

In use, the head portion can be forced in a first direction through the open neck portion into the locating portion to provide a connected configuration where contact between the head portion and edges of the open neck portion prevents removal of the head portion without application of force in an opposite direction.

The maximum width of the locating portion may correspond to the maximum width of the head portion. An outline of the first connecting arrangement may generally correspond to an outline of the second connecting arrangement. The head portion may have a bulbous outline and the locating portion may have a corresponding bulbous outline. Ends of the bulbous portions may lie in a direction parallel to a main axis of the main body (or the cap). The outline of the head (and the locating) portion may be generally horse-shoe shaped. The outline of the head (and the locating) portion may have a curved, or an inverted U-shaped, tip and side edges of the head portion may be convergent straight, or curved, lines.

In some embodiments the main body may be provided with more than one said first connecting arrangement and the cap may be provided with more than one corresponding said second connecting arrangement. In some embodiments, the main body is provided with a pair of said first connecting arrangements and the cap is provided with a corresponding pair of said second connecting arrangement. Each said first (and said second) corresponding connecting arrangements in the pair may be diametrically opposed.

The first connecting arrangement may be provided on the cap, with the open neck portion being located at a lower edge of the cap.

The second connecting arrangement may be located inboard of an end portion of the main body over which, in use, the cap is fitted. The second connecting arrangement may be formed on, or may be raised from, an outer surface of the end portion of the main body. The outer surface of the end portion of the main body may be substantially cylindrical. In use, edges of the cap and the main body adjacent to the first and the second connecting arrangements may abut when the connecting arrangements are in the connected configuration.

The first connecting arrangement may be formed as a cut-out in an outer surface of the cap. The outer surface of the cap may be substantially cylindrical.

The first direction is normally parallel to a main axis of the main body.

Whilst the invention has been described above, it extends to any inventive combination or sub-combination of novel features set out above, or in the following description or claims.

The invention may be performed in various ways and an embodiment thereof, with various modifications, will now be described by way of example only, reference being made to the accompanying drawing in which:

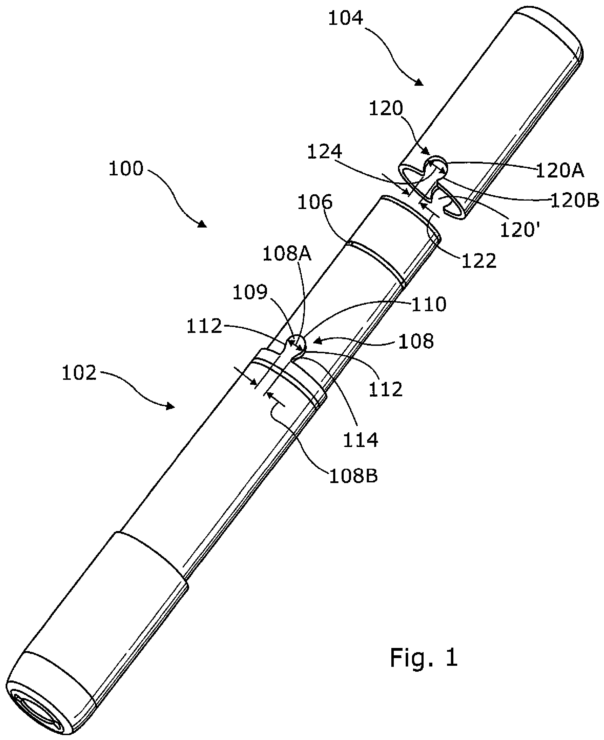

Figure 1 is a perspective view of an example autoinjector assembly.

Referring to Figure 1, an example autoinjector assembly 100 includes a main body 102 and a cap 104. The main body will contain known components of autoinjectors, such as a syringe or cartridge having a retractable needle and a drive mechanism for releasing medication via the needle on activation. Such components are well-known to the skilled person and need not be described herein in detail. Examples of autoinjectors having suitable components are ones produced by Owen Mumford Limited of Woodstock, Oxford, United Kingdom. It will also be appreciated that the arrangement described herein can also be used on other types of injection devices. Components of the autoinjector will typically be formed of a rigid plastic having a degree of resilience, such a polypropylene, polyethylene, nylon or acetol.

One end portion 106 of the body 102 is of smaller diameter than the remainder of the body and is designed to be covered by the protective cap 104. Inboard of the needle-housing end of the portion 106 there is a bulb-shaped connecting arrangement 108. The bulb-shaped connecting arrangement is formed as a raised surface at the inboard end of the portion 106, standing proud of the outer surface of that portion by around 0.5 mm - 1 mm. The connecting arrangement may be formed by any suitable method, e.g. injection moulding as an integral part of the outer surface of the body during manufacture, or it may be a separate component that is fixed to the surface. The bulb-shaped connecting arrangement has head portion 109 having a maximum width denoted by arrows 108A. The head portion has a curved (or an inverted U-shaped) tip 110 from which side edges 112 depend in a converging manner to form a waist portion 114 having a maximum width denoted by arrows 108B. The general outline of the head portion may be compared to a horse-shoe or jig-saw piece connector-like shape. The curved tip 110 and the opposite end of the bulb-shaped connecting arrangement 108 lie parallel to the main axis of the main body. Example dimensions for the widths 108A and 108B are around 5.15 mm and 4.0 mm, respectively.

The cap 104 includes a connecting arrangement 120 having an outline that generally corresponds in shape to the outline of the bulb-shaped connecting arrangement 108. The cap connecting arrangement is formed as a cut-out at the lower end of the cap. The cap connecting arrangement includes an open neck portion 122 having a maximum width denoted by arrows 120B that leads to a locating portion 124 having a maximum width that is denoted by arrows 120A. Example dimensions for the widths 120A and 120B are around 5.2 mm and 4.2 mm, respectively. In general, these maximum widths will be around (+/- 0.2 mm) 1 mm larger than the corresponding maximum widths 108A and 108B of the main body connecting arrangement. Although the cut-out in the example passes all the way through the outer wall of the cap, it will be understood that in alternative embodiments, it may only be a partial cut-out on a surface of the cap.

As can be seen, a second bulb-shaped cut-out 120' is also provided on the cap, at a location diametrically opposed to the connecting arrangement 108. A corresponding second bulb-shaped connecting arrangement (not visible) is also provided on the main body 102. In general, one or more connecting arrangement can be provided on both the main body and the cap. If there are several arrangements then they will be spaced apart around the outer surfaces of the cap and the main body at corresponding intervals.

In use, the main body 102 and the cap 104 are brought together such that the connecting arrangements are substantially aligned, as shown in Figure 1. When the head portion 109 of the main body connecting arrangement 108 contacts the edges of the open neck portion 122 of the cap connecting arrangement 120 then the user increases the force he/she is applying by hand so that the head portion pushes apart the edges of the neck portion, allowing the head portion to slide through into the locating portion 124. After the widest part of the head portion has passed through, the edges of the neck portion spring back to abut the base of the head portion. As the dimensions of the head portion and the locating portion generally correspond, contact between the (wider) head portion and the edges of the (narrower) neck portion prevents the cap from being removed, unless sufficient force is applied by the user when pulling the cap and the main body apart so as to cause the head portion to push apart the edges of the neck portion and allow the head portion to slide back out.

It will be understood that many variations to the example described above are possible. For instance, instead of the cap 104 having a connecting arrangement in the form of a cut-out, the cut-out could be provided on the main body 102, for receiving a projection of corresponding shape formed on the cap. Also, although having connecting arrangements with curved/converging side edges both above and below the portion having the maximum diameter can help the head portion be pushed in and out of the open neck portion, this need not always be the case. For instance, the connecting arrangement could comprise projections and cut-outs having arrow-like, mushroom-like shapes or the like. If more than one connecting arrangement is provided on both the cap and the main body then all of the connecting arrangements need not be identical in size/shape.

Embodiments of the autoinjector cap and main body described herein provide a visible, secure connection between the components. The connecting arrangements have no separate moving components and so are reliable and easy to manufacture.

## Claims

1. An injector assembly (100) including:
a main body (102) having a first connecting arrangement, and
a cap (104) having a second connecting arrangement for, in use, releasably connecting the cap to the first connecting arrangement of the main body,
wherein one of the first or the second connecting arrangement (120) includes a cut-out portion having an open neck portion (122) leading to a wider locating portion (124), and
**characterised in that** another of the first or the second connecting arrangement (108) includes a projection having a neck portion (114) leading to a wider head portion (109), with dimensions of the neck portion and the head portion corresponding to dimensions of the open neck portion and the locating portion so that, in use, the first and the second connecting portions releasably interlock.

2. An injector assembly (100) according to claim 1,
wherein at least part of the locating portion has a maximum width (120A) greater than a maximum width (120B) of the open neck portion (122), and
wherein the head portion (109) has a maximum width (108A) greater than the maximum width of the open neck portion (122).

3. An injector assembly (100) according to claim 1 or claim 2, where, in use, the head portion (109) is forced in a first direction through the open neck portion (122) into the locating portion (124) to provide a connected configuration where contact between the head portion and edges of the open neck portion prevents removal of the head portion without application of force in an opposite direction.

4. An injector assembly (100) according to claim 1, 2 or 3, wherein the maximum width (120A) of the locating portion (124) corresponds to the maximum width (108A) of the head portion (109).

5. An injector assembly (100) according to any one of the preceding claims, wherein an outline of the first connecting arrangement (108) generally corresponds to an outline of the second connecting arrangement (120).

6. An injector assembly (100) according to any one of the preceding claims, wherein the head portion (109) has a bulbous outline and the locating portion (124) has a corresponding bulbous outline.

7. An injector assembly (100) according to claim 6, wherein ends of the bulbous portions (109, 124) lie in a direction parallel to a main axis of the main body (102).

8. An injector assembly (100) according to claim 5, wherein the outlines of the head (109) and the locating (124) portions are generally horse-shoe shaped.

9. An injector assembly (100) according to claim 5, wherein the outlines of the head (109) and the locating (124) portions have curved tips (110) and side edges (112) that are convergent.

10. An injector assembly (100) according to any one of the preceding claims, wherein the main body (102) is provided with more than one said first connecting arrangement (108) and the cap (104) is provided with more than one corresponding said second connecting arrangement (120).

11. An injector assembly (100) according to claim 10, wherein the main body (102) is provided with a pair of said first connecting arrangements (108) and the cap (145) is provided with a corresponding pair of said second connecting arrangements (120, 120').

12. An injector assembly (100) according to claim 11, wherein each said first (108) and said second (120, 120') corresponding connecting arrangements in the pair are diametrically opposed.

13. An injector assembly (100) according to any one of the preceding claims, wherein the first connecting arrangement (120) is provided on the cap (104), with the open neck portion (122) being located at a lower edge of the cap.

14. An injector assembly (100) according to claim 13, wherein the second connecting arrangement (108) is located inboard of an end (106) of the main body (102) over which, in use, the cap (104) is fitted.

15. An injector assembly (100) according to claim 14, wherein the second connecting arrangement (108) is formed on, or is raised above, part of an outer surface of the main body (102).

16. An injector assembly (100) according to claim 14, where, in use, edges of the cap (104) and the main body (102) adjacent to the first (120) and the second (108) connecting arrangements abut when the connecting arrangements are in a connected configuration.

17. An injector assembly (100) according to any one of claims 13 to 16, wherein the first connecting arrangement (120) is formed as a cut-out in an outer surface of the cap (104).

18. An injector assembly (100) according to claim 3, wherein the first direction is parallel to a main axis of the main body (102).

19. An injector assembly (100) according to any one of the preceding claims, wherein the maximum width (108A) of the head portion (109) is around 1 mm greater than the maximum width (120A) of the open neck portion (122).

## Patentansprüche

1. Injektorbaugruppe (100), die Folgendes einschließt:
einen Hauptkörper (102), der eine erste Verbindungsanordnung aufweist, und
eine Kappe (104), die eine zweite Verbindungsanordnung aufweist, um, bei Anwendung, die Kappe lösbar mit der ersten Verbindungsanordnung des Hauptkörpers zu verbinden,
wobei eine von der ersten oder der zweiten Verbindungsanordnung (120) einen ausgeschnittenen Abschnitt einschließt, der einen offenen Halsabschnitt (122) aufweist, der zu einem breiteren Positionierungsabschnitt (124) führt, und
**dadurch gekennzeichnet, dass**
eine andere von der ersten oder der zweiten Verbindungsanordnung (108) einen Vorsprung einschließt, der einen Halsabschnitt (114) aufweist, der zu einem breiteren Kopfabschnitt (109) führt, wobei Abmessungen des Halsabschnitts und des Kopfabschnitts Abmessungen des offenen Halsabschnitts und des Positionierungsabschnitts entsprechen, so dass, bei Anwendung, der erste und der zweite Verbindungsabschnitt lösbar ineinandergreifen.

2. Injektorbaugruppe (100) nach Anspruch 1,
wobei mindestens ein Teil des Positionierungsabschnitts eine maximale Breite (120A) aufweist, die größer ist als eine maximale Breite (120b) des offenen Halsabschnitts (122), und
wobei der Kopfabschnitt (109) eine maximale Breite aufweist, die größer ist als die maximale Breite des offenen Halsabschnitts (122).

3. Injektorbaugruppe (100) nach Anspruch 1 oder Anspruch 2, wobei, bei Anwendung, der Kopfabschnitt (109) in einer ersten Richtung durch den offenen Halsabschnitt (122) in den Positionierungsabschnitt (124) gedrängt wird, um eine verbundene Konfiguration bereitzustellen, wobei eine Berührung zwischen dem Kopfabschnitt und Kanten des offenen Halsabschnitts ein Entfernen des Kopfabschnitts ohne eine Anwendung von Kraft in einer entgegengesetzten Richtung verhindert.

4. Injektorbaugruppe (100) nach Anspruch 1, 2 oder 3, wobei die maximale Breite (120A) des Positionierungsabschnitts (124) der maximalen Breite (108A) des Kopfabschnitts (109) entspricht.

5. Injektorbaugruppe (100) nach einem der vorhergehenden Ansprüche, wobei ein Umriss der ersten Verbindungsanordnung (108) im Allgemeinen einem Umriss der zweiten Verbindungsanordnung (120) entspricht.

6. Injektorbaugruppe (100) nach einem der vorhergehenden Ansprüche, wobei der Kopfabschnitt (109) einen bauchigen Umriss aufweist und der Positionierungsabschnitt (124) einen entsprechenden bauchigen Umriss aufweist.

7. Injektorbaugruppe (100) nach Anspruch 6, wobei Enden der bauchigen Abschnitte (109, 124) in einer Richtung, die parallel zu einer Hauptachse des Hauptkörpers (102) ist, liegen.

8. Injektorbaugruppe (100) nach Anspruch 5, wobei die Umrisse des Kopf- (109) und des Positionierungs- (124) -abschnitts im Allgemeinen hufeisenförmig sind.

9. Injektorbaugruppe (100) nach Anspruch 5, wobei die Umrisse des Kopf- (109) und des Positionierungs- (124) -abschnitts gekrümmte Spitzen (110) und Seitenkanten (112), die konvergent sind, aufweisen.

10. Injektorbaugruppe (100) nach einem der vorhergehenden Ansprüche, wobei der Hauptkörper (102) mit mehr als einer ersten Verbindungsanordnung (108) versehen ist und die Kappe (104) mit mehr als einer entsprechenden zweiten Verbindungsanordnung (120) versehen ist.

11. Injektorbaugruppe (100) nach Anspruch 10, wobei der Hauptkörper (102) mit einem Paar der ersten Verbindungsanordnungen (108) versehen ist und die Kappe (145) mit einem entsprechenden Paar der zweiten Verbindungsanordnungen (120, 120') versehen ist.

12. Injektorbaugruppe (100) nach Anspruch 11, wobei jede erste (108) und die zweite (120, 120') entsprechende Verbindungsanordnung in dem Paar einander diametral gegenüberliegen.

13. Injektorbaugruppe (100) nach einem der vorhergehenden Ansprüche, wobei die erste Verbindungsanordnung (120) an der Kappe (104) bereitgestellt wird, wobei der offene Halsabschnitt (122) an einer unteren Kante der Kappe angeordnet ist.

14. Injektorbaugruppe (100) nach Anspruch 13, wobei die zweite Verbindungsanordnung (108) einwärts eines Endes (106) des Hauptkörpers (102), über dem, bei Anwendung, die Kappe (104) angebracht ist, angeordnet ist.

15. Injektorbaugruppe (100) nach Anspruch 14, wobei die zweite Verbindungsanordnung (108) an einem Teil einer Außenfläche des Hauptkörpers (102) geformt ist oder oberhalb desselben erhöht ist.

16. Injektorbaugruppe (100) nach Anspruch 14, wobei, bei Anwendung, Kanten der Kappe (104) und des Hauptkörpers (102) angrenzend an die erste (120) und die zweite (108) Verbindungsanordnung aneinanderstoßen, wenn sich die Verbindungsanordnungen in einer verbundenen Konfiguration befinden.

17. Injektorbaugruppe (100) nach einem der Ansprüche 13 bis 16, wobei die erste Verbindungsanordnung (120) als ein Ausschnitt in einer Außenfläche der Kappe (104) geformt ist.

18. Injektorbaugruppe (100) nach Anspruch 3, wobei die erste Richtung parallel zu einer Hauptachse des Hauptkörpers (102) ist.

19. Injektorbaugruppe (100) nach einem der vorhergehenden Ansprüche, wobei die maximale Breite (108A) des Kopfabschnitts (109) um etwa 1 mm größer ist als die maximale Breite (120A) des offenen Halsabschnitts (122).

## Revendications

1. Ensemble d'injection (100), incluant :
un corps principal (102) comportant un premier agencement de connexion ; et
un capuchon (104) comportant un deuxième agencement de connexion pour connecter de manière amovible, en cours d'utilisation, le capuchon au premier agencement de connexion du corps principal ;
dans lequel l'un du premier ou du deuxième agencement de connexion (120) inclut une partie découpée comportant une partie de col ouverte (122) menant vers une partie de positionnement plus large (124) ; et
**caractérisé en ce que** :
un autre du premier ou du deuxième agencement de connexion (108) inclut une saillie comportant une partie de col (114) menant vers une partie de tête plus large (109), des dimensions de la partie de col et de la partie de tête correspondant à des dimensions de la partie de col ouverte et de la partie de positionnement, de sorte qu'au cours de l'utilisation, les première et deuxième parties de connexion sont mutuellement verrouillées de manière amovible.

2. Ensemble d'injection (100) selon la revendication 1,
dans lequel au moins une partie de la partie de positionnement a une largeur maximale (120A) supérieure à une largeur maximale (120B) de la partie de col ouverte (122) ; et
dans lequel la partie de tête (109) a une largeur maximale (108A) supérieure à la largeur maximale de la partie de col ouverte (122).

3. Ensemble d'injection (100) selon la revendication 1 ou 2, dans lequel, en cours d'utilisation, la partie de tête (109) est poussée de force dans une première direction à travers la partie de col ouverte (122) dans la partie de positionnement (124), pour fournir une configuration connectée, dans laquelle un contact entre la partie de tête et des bords de la partie de col ouverte empêche un retrait de la partie de tête, sans application d'une force dans une direction opposée.

4. Ensemble d'injection (100) selon les revendications 1, 2 ou 3, dans lequel la largeur maximale (120A) de la partie de positionnement (124) correspond à la largeur maximale (108A) de la partie de tête (109).

5. Ensemble d'injection (100) selon l'une quelconque des revendications précédentes, dans lequel un contour du premier agencement de connexion (108) correspond généralement à un contour du deuxième agencement de connexion (120).

6. Ensemble d'injection (100) selon l'une quelconque des revendications précédentes, dans lequel la partie de tête (109) a un contour bombé et la partie de positionnement (124) a un contour bombé correspondant.

7. Ensemble d'injection (100) selon la revendication 6, dans lequel des extrémités des parties bombées (109, 124) sont disposées dans une direction parallèle à un axe principal du corps principal (102).

8. Ensemble d'injection (100) selon la revendication 5, dans lequel les contours des parties de tête (109) et de positionnement (124) ont généralement une forme en fer à cheval.

9. Ensemble d'injection (100) selon la revendication 5, dans lequel les contours des parties de tête (109) et de positionnement (124) comportent des pointes courbées (110) et des bords latéraux (112) qui convergent.

10. Ensemble d'injection (100) selon l'une quelconque des revendications précédentes, dans lequel le corps principal (102) est équipé de plus d'un dit premier agencement de connexion (108), et le capuchon (104) est équipé de plus d'un dit deuxième agencement de connexion correspondant (120).

11. Ensemble d'injection (100) selon la revendication 10, dans lequel le corps principal (102) est équipé d'une paire desdits premiers agencements de connexion (108) et le capuchon (145) est équipé d'une paire correspondante desdits deuxièmes agencements de connexion (120, 120').

12. Ensemble d'injection (100) selon la revendication 11, dans lequel chaque dit premier (108) et chaque dit deuxième (120, 120') agencements de connexion correspondants dans la paire sont diamétralement opposés.

13. Ensemble d'injection (100) selon l'une quelconque des revendications précédentes, dans lequel le premier agencement de connexion (120) est fourni sur le capuchon (104), la partie de col ouverte (122) étant disposée au niveau d'un bord inférieur du capuchon.

14. Ensemble d'injection (100) selon la revendication 13, dans lequel le deuxième agencement de connexion (108) est disposé vers l'intérieur d'une extrémité (106) du corps principal (102) au-dessus de laquelle le capuchon (104) est ajusté en cours d'utilisation.

15. Ensemble d'injection (100) selon la revendication 14, dans lequel le deuxième agencement de connexion (108) est formé sur une partie d'une surface externe du corps principal (102) ou est surélevé au-dessus de celle-ci.

16. Ensemble d'injection (100) selon la revendication 14, dans lequel, en cours d'utilisation, des bords du capuchon (104) et du corps principal (102) adjacents aux premier (120) et deuxième (108) agencements de connexion butent les uns contre les autres lorsque les agencements de connexion se trouvent dans une configuration connectée.

17. Ensemble d'injection (100) selon l'une quelconque des revendications 13 à 16, dans lequel le premier agencement de connexion (120) est formé comme une découpe dans une surface externe du capuchon (104).

18. Ensemble d'injection (100) selon la revendication 3, dans lequel la première direction est parallèle à un axe principal du corps principal (102).

19. Ensemble d'injection (100) selon l'une quelconque des revendications précédentes, dans lequel la largeur maximale (108A) de la partie de tête (109) est supérieure d'environ 1 mm à la largeur maximale (120A) de la partie de col ouverte (122).
